# EUROPEAN PATENT APPLICATION

(11) **EP 4 303 580 A1**
(43) Date of publication of application: **10.01.2024**
(21) Application number: 22762786.6
(22) Date of filing: 13.01.2022
(51) Int. Cl.: G01N 31/00, B05B 11/00, G01N 33/493, G01N 33/52, G01N 21/78

(54) **KIT FOR MAKING ELECTRON DONOR VISIBLE AND METHOD FOR MAKING ELECTRON DONOR VISIBLE**

(30) Priority: 05.03.2021 JP 2021035299; 30.09.2021 JP 2021161274
(71) Applicant: Shachihata Inc., Aichi 4510021 (JP)
(72) Inventor: IMAI, Hideshi, Nagoya-shi, Aichi 451-0021 (JP); MINAMIDA, Norihiro, Nagoya-shi, Aichi 451-0021 (JP)
(74) Representative: Mewburn Ellis LLP
(86) International application number: PCT/JP2022/000865
(87) International publication number: WO 2022/185727

(57) **Abstract**

The present invention relates to a kit for making an electron donor visible, capable of visualizing an electron donor such as a urine component and a trace amount of protein component, and a method for making an electron donor visible using the kit. An object of the present invention is to provide a kit for making an electron donor visible and a method for making an electron donor visible, stably visualizing coloring of a site to which an electron donor is adhered over a certain period of time and causing no deterioration in appearance due to remaining of a dye. The kit for making an electron donor visible of the present invention includes a liquid A containing a coloring component including an electron donating colorant and a solvent and a liquid B containing a bleaching component including an electron acceptor and a solvent in combination. In the present invention, the liquid A and the liquid B are sprayed onto an adhesion surface of an electron donor to reveal an adhesion site of the electron donor.

## Description

### TECHNICAL FIELD

The present invention relates to a kit for making an electron donor visible, capable of visualizing a urine component and other electron donors, and a method for making an electron donor visible using the kit.

### BACKGROUND ART

Urine components are easily scattered on a floor surface and a wall surface of a bathroom, and when left to stand, the urine components are decomposed by bacteria to cause malodor such as ammonia odor. Protein components such as milk, a sweetener, a juice, and the like are easily scattered in the vicinity of a kitchen or a dining table, causing stains and odor when left to stand. It is preferable to quickly and reliably remove biologically derived stains such as saliva, vomit, and fingerprints in order to prevent malodor and infection. For these reasons, cleaning is performed periodically or as needed, but these attached substances are substantially transparent, and thus it is difficult to visually observe them after being dried. Therefore, it is not possible to confirm whether removal by cleaning can be completed, and as a result, many people feel that the odor does not disappear even after cleaning.

As a countermeasure, wall paper is replaced or an air purifier is installed, which requires a considerable cost. Therefore, in many cases, measures are taken such as simply performing ventilation or installing a fragrance or a deodorant. However, these measures do not remove the source of generation of stains and odor, and thus the effect thereof is limited.

Patent Literature 1 describes a method of spraying a pH indicator to a human or animal urine component to check health. This is a method utilizing the fact that urine components are weakly acidic, but it is not intended to visualize urine components attached to a floor surface or a wall surface.

Patent Literature 2 discloses a composition for cleaning, containing a dye that stains a protein and a surfactant. This composition for cleaning has a function of making the adhesion site of contaminants visible. However, the amount of protein contained in the urine component is significantly small in a healthy person, and thus the effect of making the urine component visible is insufficient. In addition, the dyed region is also decolorized by cleaning component, causing such a problem that coloring cannot be maintained for a certain period of time and stability of coloring cannot be secured. In addition, the dye adheres to and remains on the sprayed surface, which may deteriorate the appearance of the floor surface and the wall surface.

### CITATIONS LIST

### PATENT LITERATURE

Patent Literature 1: JP 2006-234475 A
Patent Literature 2: JP H07-504699 T

### SUMMARY OF INVENTION

### TECHNICAL PROBLEMS

An object of the present invention is to solve the above-described conventional problems, and to provide a kit for making an electron donor visible, capable of stably visualizing coloring of an adhesion site of an electron donor such as a urine component or a trace amount of a protein component over a certain period of time, and causing no deterioration in appearance due to remaining of a dye, and a method for making an electron donor visible using the kit.

### SOLUTIONS TO PROBLEMS

A kit for making an electron donor visible of the present invention made to solve the above problems combines a liquid A containing a coloring component including an electron donating colorant and a solvent and a liquid B containing a bleaching component including an electron acceptor and a solvent.

The electron donor is preferably a compound containing at least one nucleophilic atomic group, and the nucleophilic atomic group is preferably any one selected from the group consisting of C=C, C-N, C=N, -NH₂, -NH-, - SH, and combinations thereof.

The liquid A or the liquid B preferably contains a quaternary ammonium salt, and the liquid A or the liquid B preferably contains a thickener to be gelled by calcium ions.

In addition, in a method for making an electron donor visible of the present invention made to solve the above problems, a liquid A containing a coloring component including an electron donating colorant and a solvent and a liquid B containing a bleaching component including an electron acceptor and a solvent are sprayed onto an adhesion surface of the electron donor to reveal an adhesion site of the electron donor.

The electron donor is preferably a compound containing at least one nucleophilic atomic group, and the nucleophilic atomic group is preferably any one selected from the group consisting of C=C, C-N, C=N, -NH₂, -NH-, - SH, and combinations thereof.

### ADVANTAGEOUS EFFECTS OF INVENTION

In order to make an electron donor visible according to the present invention, first, a liquid B containing a bleaching component including an electron acceptor and a solvent is sprayed onto the adhesion surface of the electron donor. The bleaching component reacts with the electron donor at the adhesion site of the electron donor, and the bleaching ability is reduced accordingly, but the bleaching ability is not reduced at other sites. Then, when a liquid A containing a coloring component including an electron donating colorant and a solvent is sprayed thereon, the bleaching ability has reduced at the adhesion site of the electron donor, and thus the coloring component is not decolorized. However, the bleaching ability has not deteriorated in other sites, and thus the coloring component is decolorized and becomes colorless. The liquid B and the liquid A may be sprayed almost simultaneously, or the liquid A may be sprayed prior to the liquid B.

As a result, the dye remains only at the adhesion site of the electron donor, and the dye at sites to which the electron donor does not adhere is decolorized and becomes colorless, and thus it is possible to stably reveal only the adhesion site of the electron donor over a certain period of time. Further, according to the present invention, the coloring component sprayed to a site other than the adhesion site of the electron donor becomes colorless, thus providing such an advantage that the appearance of the sprayed surface is not deteriorated. The quaternary ammonium salt is contained in the liquid A or the liquid B, thereby providing such an advantage that the decoloring speed of the liquid A due to the reaction between the liquid A and the liquid B can be increased.

In addition, the kit for making an electron donor visible of the present invention is a combination of these liquid A and liquid B, and thus it is possible to optimize the respective concentrations and sell the kit, and it is possible to prevent discoloration of wallpaper or the like due to excessive decolorization or to prevent remaining of a dye due to insufficient decolorization. In particular, it is convenient for home use if these are filled in a spray container for sale.

### BRIEF DESCRIPTION OF DRAWINGS

Fig. 1 is a cross-sectional view showing an example of a kit for making an electron donor visible of the present invention.
Fig. 2 is an explanatory view showing a function of a quaternary ammonium salt in the present invention.
Fig. 3 is a diagram illustrating a method for using the kit for making an electron donor visible of the present invention.

### DESCRIPTION OF EMBODIMENTS

Preferred embodiments of the present invention will be described below.

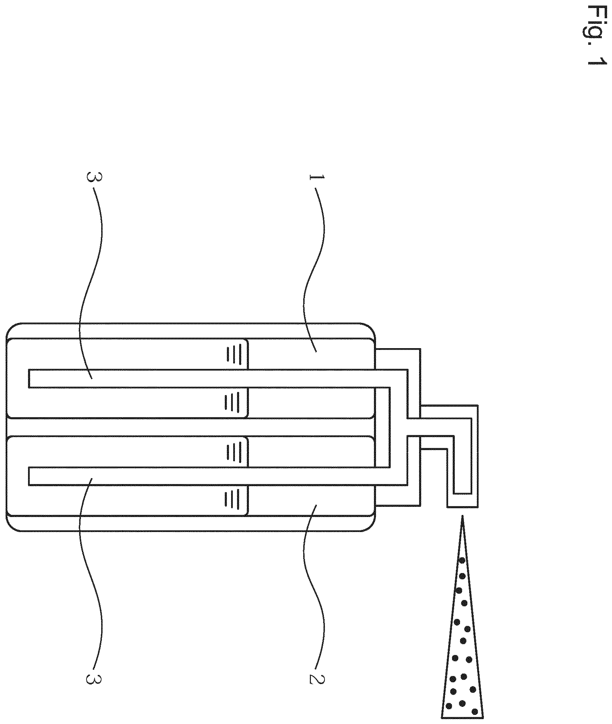

In the present invention, a liquid A containing a coloring component including an electron donating colorant and a solvent and a liquid B containing a bleaching component including an electron acceptor and a solvent are used in combination. The liquid A and the liquid B are preferably sold as a kit in which the liquid A and the liquid B are filled in different spray containers. The kit for making an electron donor visible in the present invention refers to a tool used for spraying the liquid A and the liquid B to an electron donor to make the electron donor visible, and as its form, for example, one or more spray containers, packs, collecting tubes, syringes, and the like filled with the liquid A and the liquid B, respectively, can be considered, but the form is not limited. In addition, it may be configured to be electrically driven using a sensor or power supply, or it may be configured to be like an aerosol in which contents are sprayed in a mist form by the pressure of gas. Further, it is preferable that the liquid A and the liquid B are filled in different spray containers. The form in which different spray containers are filled is not necessarily required to be two containers physically separated from each other, and may be a form in which individual first and second containers are connected. In addition, as shown in Fig. 1, it may be configured to connect the first and second containers, and mix different types of liquids before spraying and spray the mixed liquid from one injection port by a suction pipe 3 to which both containers 1 and 2 are connected. In addition, the above spray container may be configured to be electrically driven using a sensor or power supply, or it may be configured to be like an aerosol in which contents are sprayed in a mist form by the pressure of gas.

The electron donor to be visualized is preferably a compound containing at least one nucleophilic atomic group. In addition, the nucleophilic atomic group is preferably any one selected from the group consisting of C=C, C-N, C=N, -NH₂, -NH-, -SH, and combinations thereof.

Specific examples of the electron donor include amino acids, proteins and peptides including amino acids, creatinine, carbohydrates, vitamin C, amylase, ethanol, and ammonia. Amino groups of amino acids and ether groups of glucose or dextrose are electron donors that are easily oxidized, and urine components also contain proteins, and thus can be visualized. In addition, compounds having a binding site (5-) with high electron density, such as C=C, C=N, C-N (including a peptide bond), -NH2, -NH-, and -SH, are easily oxidized. Therefore, it is also possible to visualize compounds such as starch, proteins, amino acids, and sugars in addition to urine components. This makes it possible to visualize fingerprints, sweat, biofilms, and the like containing urea and proteins in addition to urine components, saliva, and vomit. In addition, sweeteners and juices including glucose or dextrose, soft beverages and teas including antioxidants and vitamin C, foods such as sake, rice, and sugar, soap scum, and the like can also be visualized.

The liquid A is a liquid containing a coloring component including an electron donating colorant and a solvent. The electron donating colorant is a colorant that loses electrons and is easily oxidized. This property applies to all dyes, and dyes shown in Tables 1, 2, and 3 below can be used. Table 1 shows acid dyes including anthraquinone-based dyes, Table 2 shows basic dyes, and Table 3 shows other dyes. C.I in the tables is a color index defined by the societies of dyers in the United Kingdom and the United States. The amount added is preferably about 10 ppm to 10% by weight, more preferably 100 ppm to 1% by weight as long as the solvent is colored.

**[Table 1]**

| Acidic dye name | C.I | Product name: Manufacturer |
|---|---|---|
| Nitroso-based dye | Acid Green 1 | Acid Green: R.A.Dyestuffs(I) |
| Nitro-based dye | Acid Yellow 24 | Martius Yellow: FUJIFILM Wako Pure Chemical Corporation |
| Monoazo-based dye | Acid Red 18 | WATER RED1: Orient Chemical Industries Co., Ltd. |
| Diazo-based dye | Acid Blue 113 | Suminol Milling Granine 5R(N) |
| Triphenylmethane-based dye | Acid Blue 1 | WATER BLUE 106: Orient Chemical Industries Co., Ltd. |
| Xanthene-based dye | Acid Red 52 | WATER RED 27: Orient Chemical Industries Co., Ltd. |
| Anthraquinone-based dye | Acid Blue 112 | |
| Anthraquinone-based dye | Acid Blue 40 | Aminyl Blue E-2 GL: Taoka Chemical Co., Ltd. |
| Indigoid-based dye | Acid Blue 74 | Food Blue 2 |
| Aminoketone-based dye | Acid Yellow 7 | Brilliant sulfaflavine: MP Biomedicals, Inc. |

**[Table 2]**

| Basic dye name | C.I | Product name: Manufacturer |
|---|---|---|
| Diphenylmethane-based dye | Basic Yellow 2 | Auramine: FUJIFILM Wako Pure Chemical Corporation |
| Acridine-based dye | Basic Orange 14 | Acridine orange: FUJIFILM Wako Pure Chemical Corporation |
| Methine-based dye | Basic Yellow 23 | Basic Yellow 28 Cathilon S.W.T Yellow-3 Nissei Kasei Co., Ltd. |
| Thiazole-based dye | Basic Yellow 1 | Basic Yellow 1: FUJIFILM Wako Pure Chemical Corporation |
| Azine-based dye | Basic Red 5 | Neutral red: FUJIFILM Wako Pure Chemical Corporation |
| Thiazine-based dye | - | Methylene blue tetrahydrate: FUJIFILM Wako Pure Chemical Corporation |

**[Table 3]**

| Other dye name | C.I | Product name: Manufacturer |
|---|---|---|
| Triazo-based dye | Direct Black 38 | Chlorazol Black E: Tokyo Chemical Industry Co., Ltd. |
| Polyazo-based dye | Direct Red 80 | Sirius red: FUJIFILM Wako Pure Chemical Corporation |
| Quinoline dye | Solvent Yellow 33 | Quinoline Yellow S: Toronto Research Chemicals Inc. |
| Oxazine dye | Mordant Blue 10 | Gallocyanine: Alfa Aesar |
| Carotenoid-based dye | Food Orange 5 | β-carotene: FUJIFILM Wako Pure Chemical Corporation |
| Indophenol-based dye | None | Indophenol blue: FUJIFILM Wako Pure Chemical Corporation |
| Hydroxyketone-based dye | None | 2,3,4-Trihydroxybenzophenol Indophenol Blue: Tokyo Chemical Industry Co., Ltd. |
| Anthocyanin-based dye | - | Red Color DC: Mitsubishi Chemical Foods Corporation |
| Alizarin Red S | - | Alizarin Red S solution: Junsei Chemical Co., Ltd. |
| Bromothymol Blue | - | Bromothymol Blue solution: Junsei Chemical Co., Ltd. |

As the solvent, water such as ion-exchanged water, pure water, or tap water, or an organic solvent can be used. As the organic solvent, alcohols such as methanol, ethanol, propanol, butanol, pentanol, hexanol, and benzyl alcohol, diols or triols such as glycerin, diglycerin, triglycerin, ethylene glycol, diethylene glycol, triethylene glycol, propylene glycol, and polyethylene glycol can be used singly or in combination. The amount added is desirably 60 to 99.99% by weight.

The liquid A or the liquid B can contain a quaternary ammonium salt as an erasure aid. Examples of the quaternary ammonium salt include tetramethylammonium chloride, tetrabutylammonium chloride, methylbenzethonium chloride, distearyldimethylammonium chloride, cetylpyridinium chloride, alkyltrimethylammonium chloride, octyltrimethylammonium chloride, decyltrimethylammonium chloride, dodecyldimethylbenzylammonium chloride, dodecyltrimethylammonium chloride, tetradecyltrimethylammonium chloride, hexadecyltrimethylammonium chloride, cetyltrimethylammonium chloride, stearyltrimethylammonium chloride, benzyltrimethylammonium chloride, benzyltriethylammonium chloride, dialkyldimethylammonium chloride, tetramethylammonium hydroxide, benzalkonium bromide, cetrimonium bromide, domiphen bromide, alkyltrimethylammonium bromide, hexadecyltrimethylammonium bromide, and hexadecyltrimethylammonium bromide, and are not limited thereto. Preferably, benzalkonium chloride, benzethonium chloride, and dodecyltrimethylammonium chloride can be used. Further, a thickener such as synthetic hectorite can be added to enhance the adhesion. The erasure aid is preferably added in an amount of 10 ppm to 10% by weight.

As shown in the data of Examples described later, when a quaternary ammonium salt that is a cationic surfactant is contained as the erasure aid, the electron donating colorant sprayed on the surface to which the electron donor does not adhere rapidly decolors. The action mechanism of the quaternary ammonium salt in the present invention is considered as follows.

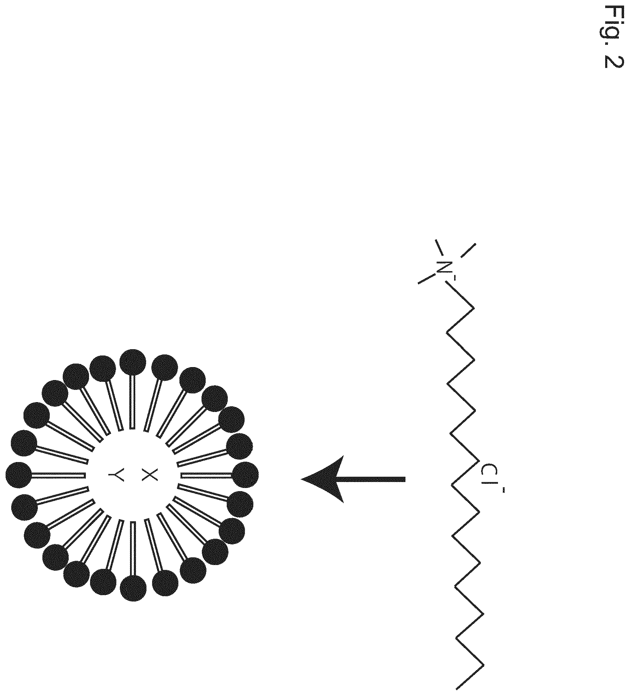

The quaternary ammonium salt cation shown in the upper part of Fig. 2 has a hydrophilic group and a hydrophobic group at both ends of the molecule, and forms micelles in which the outer side is hydrophilic groups and the inner side is hydrophobic groups as shown in the lower part of Fig. 2. A dye X and a bleaching component Y are incorporated into the micelle, and local concentration is performed to promote bleaching.

Further, a known thickener can be added to the liquid A or the liquid B to enhance the adhesion. Examples of the known thickener include water-swellable silicate particles, alginate, carboxymethylcellulose, gum arabic, arginine polymer, sodium alginate, algin propylene glycol, ethyl cellulose, xanthan gum, carrageenan, pectin, gellan gum, methyl cellulose, polyvinylpyrrolidone resin, butyral resin, acrylic resin, and cellulose nanofiber. In particular, the thickener added to the liquid A or the liquid B is desirably a thickener that is gelled by calcium ions. Examples of the thickener gelled by calcium ions include water-swellable silicate particles, alginate, carrageenan, LM pectin, and LA gellan gum. Since the thickener gelled by calcium ions reacts with calcium ions contained in the urine component to be gelled, adhesion to the urine component is particularly enhanced, and for example, liquid dripping is prevented when a liquid is sprayed on the urine component adhering to a wall surface. Further, the water-swellable silicate particles have high thixotropy, and exhibit an effect of significantly reducing liquid viscosity by the pressure applied when the liquid passes through the micropores of the nozzle during spraying, and increasing the liquid diffusion during spraying, which is more desirable. The amount added is preferably 0.1 to 20% by weight.

The term "water-swellable silicate particles" is a generic term of the group consisting of smectite, bentonite, vermiculite, and mica, and the term "smectite" is a generic term of the group consisting of montmorillonite, which is a di-octahedral hydrated laminar silicate mineral, hyderite, nontronite, saponite, hectorite, sauconite, and stevensite, which are similar in structure to montmorillonite. The water-swellable silicate particles may be a natural product or a synthetic product.

The alginate is not particularly limited as long as it is a salt of alginic acid, but is preferably a monovalent cation salt of alginic acid, and is, for example, a sodium salt (sodium alginate), a potassium salt (potassium alginate), or an ammonium salt (ammonium alginate) of alginic acid.

The liquid B is a liquid containing a bleaching component including an electron acceptor and a solvent. The electron acceptor is an oxidizing agent that oxidizes a counterpart substance by receiving electrons, and can be selected from the group consisting of metal hypochlorite such as sodium hypochlorite, metal chlorite, hydrogen peroxide, metal perborate, metal percarbonate, metal peroxide, acyl peroxide, benzoyl peroxide, peracetic acid, ozone, sodium bisulfate, nitrogen dioxide, chlorine, chlorine dioxide, azodicarbonamide, sodium sulfite, sodium metabisulfite, percarbonate, tetraacetylene ethylenediamine, metal peracid monosulfate, and mixtures thereof. The amount of the electron acceptor added is preferably 10 ppm to 20% by weight, and more preferably 100 ppm to 6% by weight. Further, a thickener such as synthetic smectite can also be added to the liquid B to enhance the adhesion. As the thickener contained in the liquid B, the above-described thickener can be used, and the amount added is preferably 0.01 to 20% by weight.

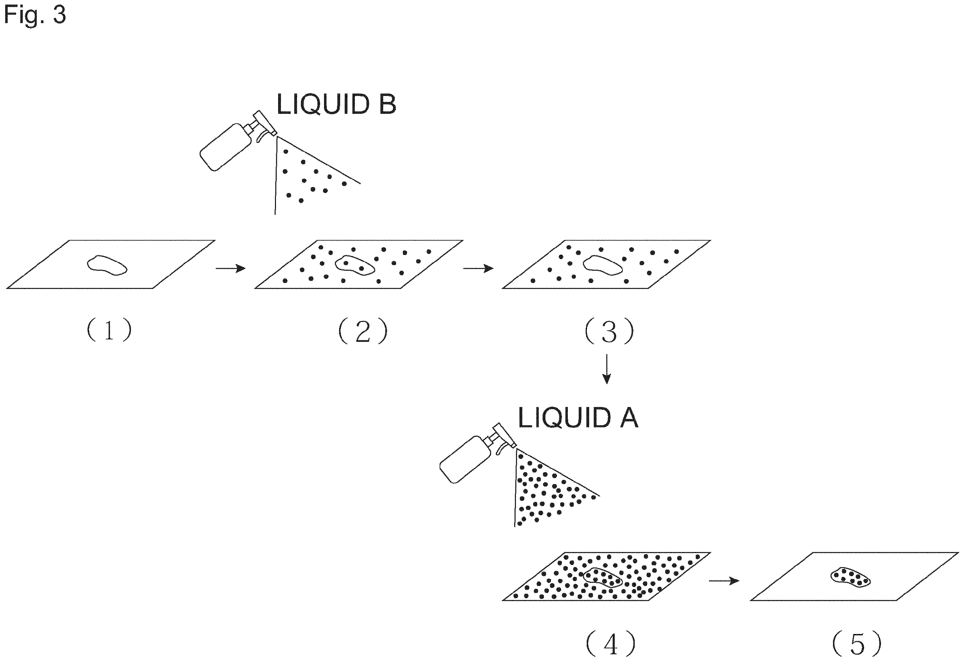

The liquid A and the liquid B described above are filled in different containers, respectively, to form a kit for making an electron donor visible. The containers are preferably spray containers. An example of a method for using the kit for making an electron donor visible of the present invention will be described below with reference to Fig. 3.

Fig. 3 (1) is a view showing an adhesion surface to which a urine component is adhered. Herein, the adhesion site of the urine component is surrounded by a line, but it is not actually visible. In general, human or animal urine components include urea, creatinine, sodium hydrogen phosphate, ammonium chloride, sodium nitrite, potassium phosphate, calcium ions, and the like.

As shown in Fig. 3 (2), the liquid B containing a bleaching component including an electron acceptor and a solvent is sprayed onto the adhesion surface. The bleaching component in the liquid B reacts with the urine component at the adhesion site of the urine component, and the bleaching ability is reduced accordingly. However, the bleaching ability is not deteriorated in other sites. This state is shown in (3). Then, as shown in (4), the liquid A containing a coloring component including an electron donating colorant and a solvent is sprayed thereon. The bleaching ability of the liquid B previously sprayed is deteriorated at the adhesion site of the urine component, and thus the coloring component is not decolorized and is colored by the liquid A, but the bleaching ability of the liquid B is not deteriorated at other sites, and thus the coloring component of the liquid A is decolorized and becomes colorless.

As a result, as shown in Fig. 3 (5), the adhesion site of the urine component is colored in a state where the dye of the liquid A remains, and the dye of the liquid A is decolorized in the other sites. Therefore, it is possible to reveal and visually recognize the adhesion site of the urine component, and it is possible to reliably remove the adhered urine component by cleaning the adhesion site. In addition, the dye of the liquid A is decolorized at a site other than the adhesion site of the urine component, and thus it is possible to prevent deterioration of appearance due to remaining of the dye of the liquid A. When the quaternary ammonium salt serving as an erasure aid is contained in the liquid A or the liquid B, the decoloring reaction is accelerated, and the decoloring speed of the liquid A due to the reaction between the liquid A and the liquid B can be increased.

In Fig. 3, the liquid A is sprayed after the liquid B is sprayed, but the liquid B and the liquid A may be sprayed almost simultaneously, or the liquid A may be sprayed prior to the liquid B. However, in order to more reliably progress the reaction between the urine component and the liquid B, it is preferable to spray the liquid B and the liquid A in this order as shown in Fig. 3.

### [Examples]

As shown in Tables 4 and 5, the liquid A and the liquid B were prepared and filled in spray containers, respectively. The solvents in both the liquid A and the liquid B were ion-exchanged water. The liquid A and the liquid B were sequentially sprayed onto wall surface tile that has been dried after being adhered to the target substance, and the visibility of the adhesion site of the target component, the decoloring speed of the non-adhesion site, the colorability of the non-adhesion site, the adhesion to the wall surface, and the diffusibility of the sprayed liquid were evaluated and described in Tables 4 and 5. As "urine" among the target components, artificial urine containing urea, ammonium chloride, and calcium ions was used. Among the target components, "commercially available drinking water (tea)" contains vitamin C as an antioxidant component.

With respect to the evaluation criteria in Tables 4 and 5, the visibility refers to the visibility of the adhesion site of the target component. In comparison between the adhesion site and the not-adhesion site, the case where the adhesion site of the target component was colored by a colorant, the adhesion site was visually clearly visible was evaluated as o, and the case where the boundary between the adhesion site and the not-adhesion site was ambiguous and was difficult to visually recognize was evaluated as Δ or ×.

In addition, the decoloring speed other than the dirt adhesion site refers to a speed at which the coloring component of the liquid A adhered to the site other than the adhesion site was decolored by the bleaching component of the liquid B. The case where decoloring was remarkably fast within 30 seconds was evaluated as ⊚, the case where decoloring was fast within 1 minute was evaluated as o, and the case where it took more than 1 minute was evaluated as Δ or ×.

Further, the colorability other than dirt refers to the colorability of the site other than the adhesion site, and the case where the site other than the adhesion site was not colored by spraying the liquid A and the liquid B was evaluated as o, whereas the case where the site was colored was evaluated as ×.

In addition, the adhesion to the wall refers to a state of the sprayed liquid when the target substance is adhered to the wall and the liquid to be sprayed of the liquid A and the liquid B is sprayed to the target component adhered to the wall. A case where the sprayed liquid hardly moved from the adhesion site of the target component without dripping was evaluated as ⊚. The case where the sprayed liquid slightly dipped and slightly moved from the adhesion site of the target component, but the degree was small and the sprayed liquid could be easily wiped off was evaluated as o. The case where the sprayed liquid dripped and moved from the adhesion site of the target component and it was difficult to wipe off the sprayed liquid was evaluated as Δ or ×.

Further, with respect to the diffusibility of the sprayed liquid, the diffusibility of the liquid was evaluated when the liquid was sprayed by the spray container to the target substance adhered to the tile and dried. The case where the liquid diffused to the extent of sufficiently covering the adhesion site of the target component was evaluated as ⊚, the case where the liquid diffused to the extent of covering the adhesion site of the target component was evaluated as o, and the case where the liquid did not sufficiently diffuse and the adhesion site of the target component could not be covered was evaluated as Δ or ×.Spray diffusibility was evaluated in each of a spray container of the liquid A and a spray container of the liquid B.

In Examples 1 to 39, the evaluation was excellent with ⊚ or o, but with respect to the visibility of Comparative Examples 1 to 3, in which the liquid B was not used, the boundary between the adhesion site and the site other than the adhesion site was ambiguous, resulting in poor visibility. In addition, for the colorability other than dirt, the site other than the adhesion site was also colored, resulting in ×. In addition, in Comparative Example 4, in which acetic acid having no electron donating atomic group was used as the target substance, the target component could not be visualized.

As found from these Examples, various dyes can be used as the electron donating colorant of the liquid A. As the electron acceptor of the liquid B, an oxidizing agent other than sodium hypochlorite can be used.

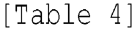

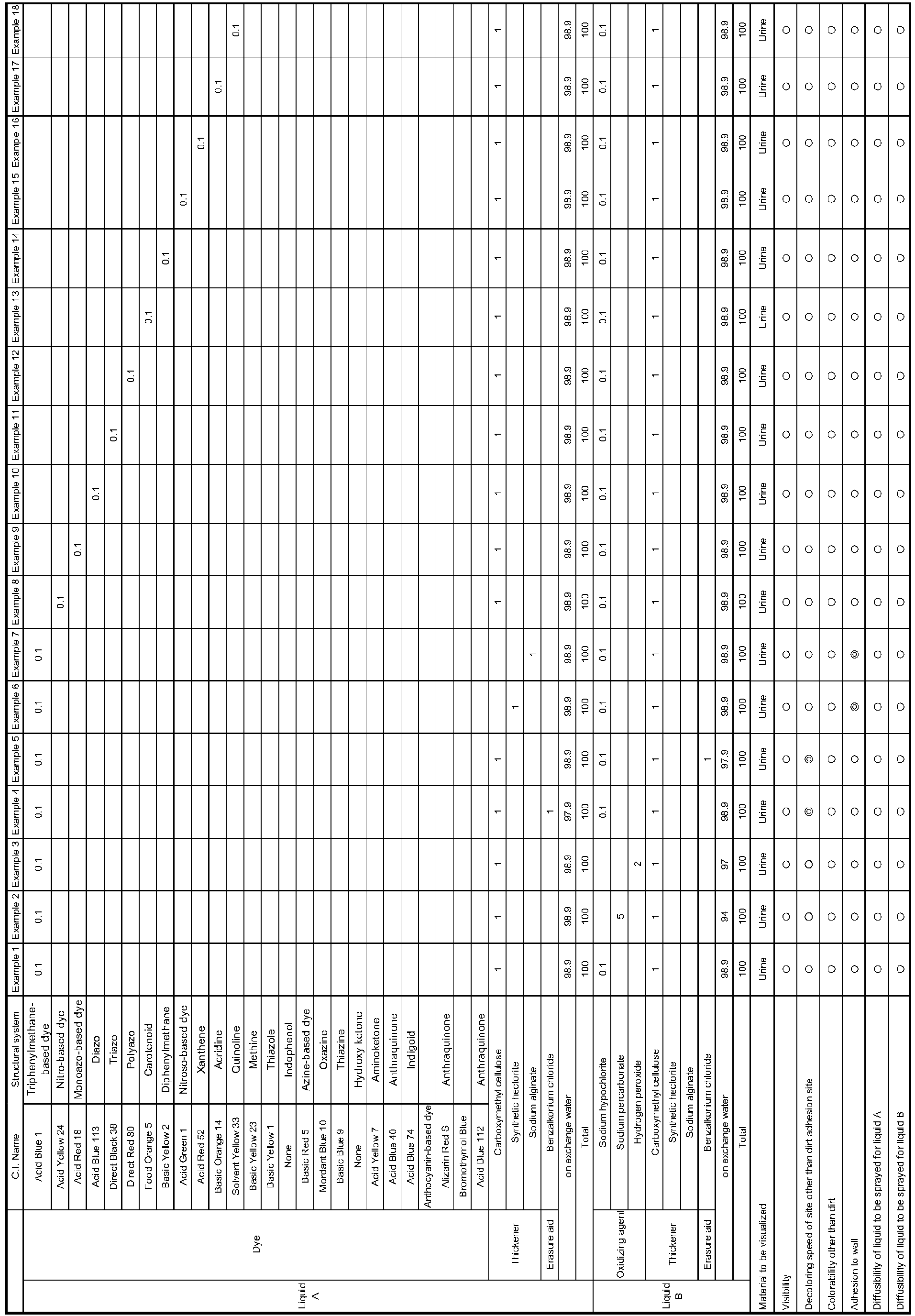

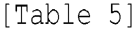

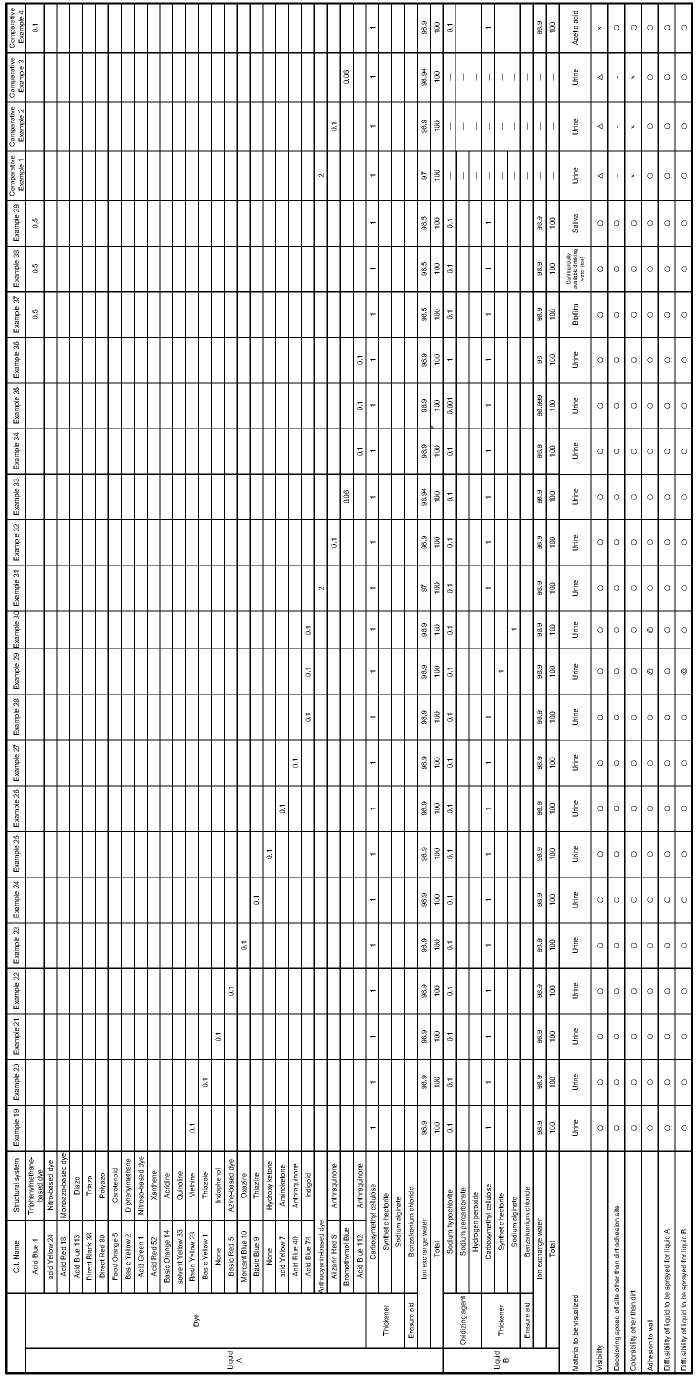

### REFERENCE SIGNS LIST

- 1: First container
- 2: Second container
- 3: Suction pipe

## Claims

1. A kit for making an electron donor visible, comprising: a liquid A containing a coloring component including an electron donating colorant and a solvent; and a liquid B containing a bleaching component including an electron acceptor and a solvent, in combination.

2. The kit for making an electron donor visible according to claim 1, wherein the electron donor is a compound containing at least one nucleophilic atomic group.

3. The kit for making an electron donor visible according to claim 2, wherein the nucleophilic atomic group is any one selected from the group consisting of C=C, C-N, C=N, -NH₂, -NH-, -SH, and combinations thereof.

4. The kit for making an electron donor visible according to any one of claims 1 to 3, wherein the liquid A or the liquid B contains a quaternary ammonium salt.

5. The kit for making an electron donor visible according to any one of claims 1 to 4, wherein the liquid A or the liquid B contains a thickener to be gelled by calcium ions.

6. The kit for making an electron donor visible according to any one of claims 1 to 5, wherein the liquid A and the liquid B are filled in different spray containers, respectively.

7. A method for making an electron donor visible, the method comprising spraying a liquid A containing a coloring component including an electron donating colorant and a solvent and a liquid B containing a bleaching component including an electron acceptor and a solvent onto an adhesion surface of an electron donor to reveal an adhesion site of the electron donor.

8. The method for making an electron donor visible according to claim 7, wherein the electron donor is a compound containing at least one nucleophilic atomic group.

9. The method for making an electron donor visible according to claim 8, wherein the nucleophilic atomic group is any one selected from the group consisting of C=C, C-N, C=N, -NH₂, -NH-, -SH, and combinations thereof.
